# EUROPEAN PATENT APPLICATION

(11) **EP 1 987 803 A2**
(43) Date of publication of application: **05.11.2008**
(21) Application number: 08445018.8
(22) Date of filing: 30.04.2008
(51) Int. Cl.: A61F 2/08, A61B 17/04

(54) **Expanding plug for tendon fixation**

(30) Priority: 01.05.2007 US 915287 P
(71) Applicant: Arthrex, Inc., Naples, Florida 34108-1945 (US)
(72) Inventor: Saphiro, Aprihl, Fort Myers, Florida 33967-3633 (US)
(74) Representative: Modin, Jan

(57) **Abstract**

An expanding plug for tendon fixation features two resilient arms configured to expand radially to achieve interference fixation of a graft tendon inside of a bone tunnel. The tendon graft is assembled to the expanding bolt and situated within a bone tunnel. Passing suture is used to pull the expanding plug into the tunnel in a first direction. Pulling the expanding plug in a second direction (which is opposite the first direction) deploys the resilient arms and places the expanding plug in the deployed configuration.

## Description

### BACKGROUND OF THE INVENTION

### 1. Field of the Invention:

The present invention relates to apparatus and methods for surgically anchoring replacement tendon constructs.

### 2. Description of the Related Art:

When soft tissue such as a ligament or a tendon becomes detached from a bone, surgery is usually required to reattach or reconstruct the tissue. Often, a tissue graft is attached to the bone to facilitate regrowth and permanent attachment. Various fixation devices, including sutures, screws, staples, wedges, and plugs have been used in the past to secure soft tissue to bone. In typical interference screw fixation, for example, the graft is fixed to the bone by driving the screw into a blind hole or a tunnel in the bone while trapping the end of the graft between the screw and the bone tunnel. In other methods, the graft is simply pinned against the bone using staples or sutures tied around the end of the graft to the bone.

A need exists for secure and simplified methods and devices for anchoring tendon grafts.

### SUMMARY OF THE INVENTION

The present invention overcomes disadvantages of the prior art, such as those noted above, by providing an expanding device or bolt for soft tissue fixation (for example, for tendon fixation). The expanding device or bolt is a flexible "A-Frame" that is configured to collapse when pushed in a first direction (for example, when pushed antegrade) within a tunnel or socket, and to expand and engage adjacent tissue (for example, bone of the tunnel or socket) when pulled in a second direction (for example, when pulled retrograde). Fixation devices of the flexible "A-Frame" device (for example, two arms of resilient material) are configured to expand diametrically to achieve interference fixation of soft tissue (for example, graft tendon) in a bone tunnel.

The present invention also provides a graft fixation surgical method employing an expanding device, comprising the steps of: (i) pre-forming a hole or tunnel (for example, a femoral or tibial tunnel); (ii) attaching a graft (for example, a soft tissue graft) to the expanding device or expanding bolt to form a graft/expanding device assembly; (iii) inserting the graft/expanding device assembly into the tunnel in an antegrade manner, so that the expanding device is in a collapsed state; and (iv) pulling the graft/expanding device assembly in a retrograde manner, so that the expanding device expands (in the fully deployed configuration) and engages bone to resist pullout.

Other features and advantages of the present invention will become apparent from the following description of the invention, which refers to the accompanying drawings.

### BRIEF DESCRIPTION OF THE DRAWINGS

FIGS. 1(a)-(f) illustrate various views of a fixation device according to the present invention;

FIG. 2 is a perspective view of the fixation device of the present invention;

FIG. 3 is another perspective of the fixation device of the present invention; and

FIG. 4 illustrates a graft tendon assembled to a fixation device according to the present invention.

### DETAILED DESCRIPTION OF THE PREFERRED EMBODIMENTS

In the following detailed description, reference is made to various specific embodiments in which the invention may be practiced. These embodiments are described with sufficient detail to enable those skilled in the art to practice the invention, and it is to be understood that other embodiments may be employed, and that structural and logical changes may be made without departing from the spirit or scope of the present invention.

The expanded device (bolt) of the present invention is configured to expand diametrically to achieve interference fixation of a graft tendon in a bone tunnel. The graft (for example, tendon graft) is assembled to the expanding bolt and then situated within a bone tunnel. A passing strand (for example, suture) is used to pull a joint-line end of the expansion bolt into the tunnel in the collapsed state in a first direction. When pulled in a different direction (for example, when pulled retrograde), the fixation devices is configured to expand radially to achieve interference fixation of soft tissue (for example, graft tendon) in a bone tunnel.

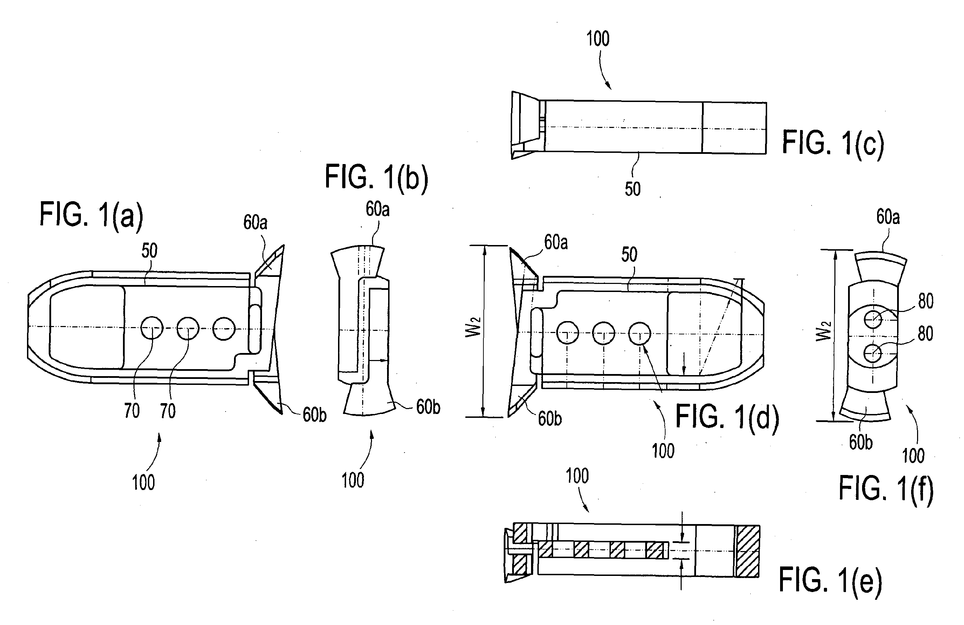

Referring now to the drawings, where like elements are designated by like reference numerals, an exemplary expanding device 100 of the present invention is illustrated in FIGS. 1-4. Expanding device or bolt 100 comprises a body 50 in the shape of a flexible "A-Frame" that is configured to collapse when pushed in a first direction (for example, when pushed antegrade) within a tunnel or socket, and to expand and engage adjacent tissue (for example, bone of the tunnel or socket) when pulled in a second direction (for example, when pulled retrograde and in an axial direction).

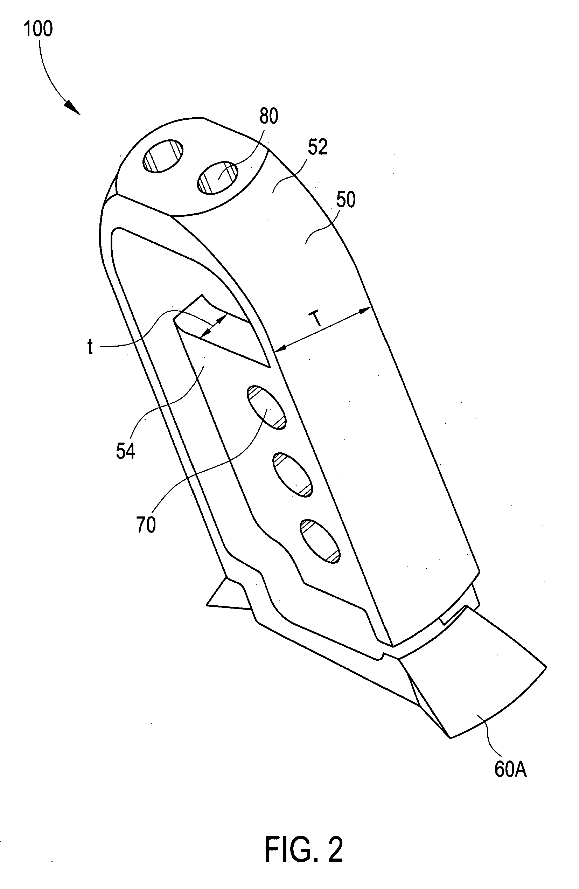

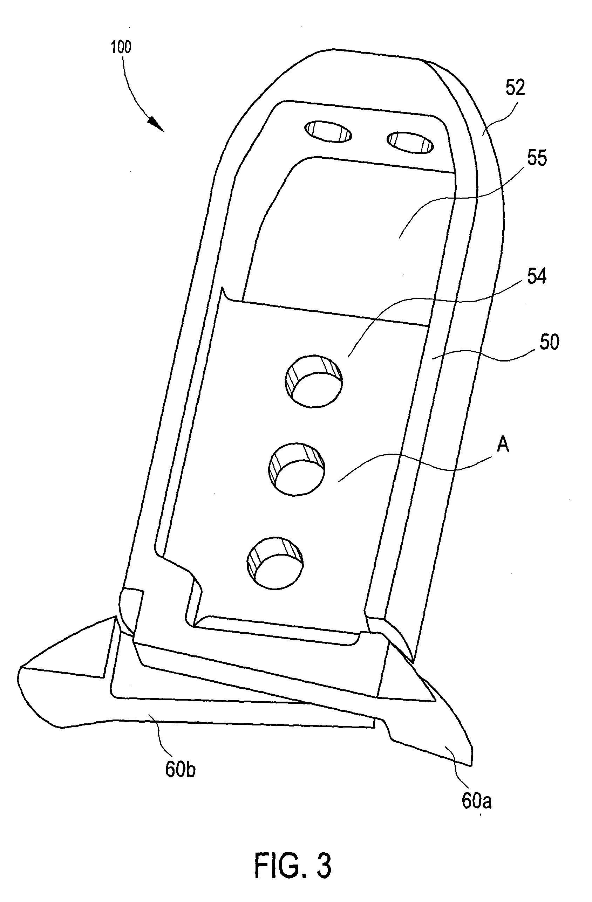

As shown in FIGS. 2 and 3, body 50 of the expanding device 100 comprises a main elongated frame 52 having a generally rectangular configuration, and an inner part 54 also with a generally rectangular configuration and located fully within the perimeter of the main frame 52. The surface area A (FIG. 3) of inner part 54 is about 2/3 smaller than that of the main frame 52, to reduce the amount of material (for example, PLLA) employed for the formation of the device 100 and to permit secure fixation of the free ends of a ligament graft that is to be seated within the sides of the inner part 54 and/or within window or aperture 55. The thickness "t" (FIG. 2) of the inner part 54 is smaller than the thickness "T" (FIG. 2) of the main frame 52 (for example, about half the thickness "T"), also to allow enhanced seating of the free ends of a graft ligament when used in conjunction with the fixation device.

A plurality of through holes 70 are provided within the inner part 54 (extending from un upper surface to a lower surface of the inner part 54) to allow a secure suturing to the graft and bone growth infiltration. Preferably, and as described below, the expanding device 100 is sutured to a graft (for example, a tendon or ligament graft) with multiple holes to fixate the graft free ends. The multiple holes 70 (three in the exemplary embodiment shown in FIGS. 2 and 3) also reduce the amount of material (for example, PLLA) employed for the formation of the device 100 and allow accelerated tissue/bone ingrowth.

Flexible "A-Frame" device 100 comprises fixation devices 60a, 60b that are configured to expand diametrically and radially to achieve interference fixation of soft tissue (for example, graft tendon) in a bone tunnel. In an exemplary embodiment, fixation devices 60a, 60b comprise a plurality of arms or flanges (for example, two arms as shown in FIGS. 1-3) formed of resilient material, that allows the arms to expand and engage tissue. In an exemplary embodiment, each of the fixation devices 60a, 60b has a width which is about equal to the width or thickness "T" of the main frame 52. The arms are configured to expand radially and to increase the width W1 of the bolt (corresponding to the collapsed state) to a width W2 (corresponding to the expanded state) (FIGS. 1(d) and 1(f)).

Although body 50 and fixation devices 60 of the "A-Frame" device 100 have the profile and configuration shown in FIGS. 1-3, these features may have various configurations that allow the device to be maneuvered axially in a first direction, and to spread open and expand when urged in a second (for example, an opposite) direction. Additional fixation can be provided using suture.

Expanding devices or anchors according to the present invention can be used for arthroscopic procedures such as ligament repairs. The devices are also advantageous for open and mini-open surgical procedures. Specific examples of applicable procedures include cortical bone-soft tissue fixation, Bankart and SLAP shoulder repairs.

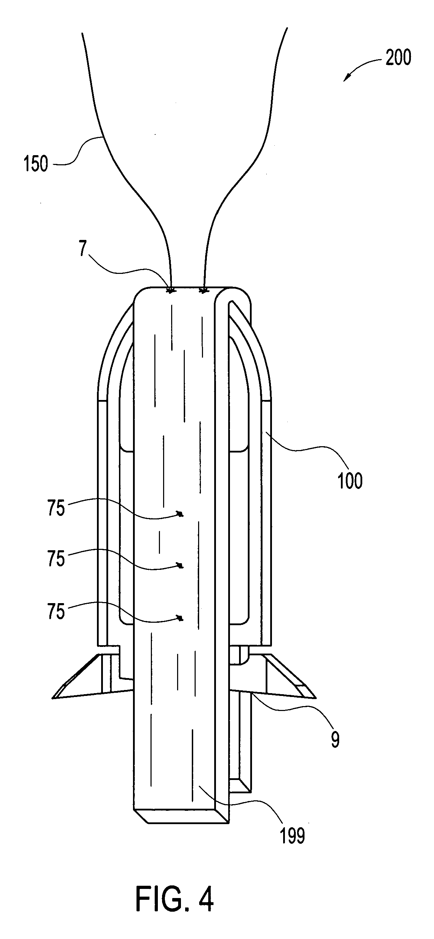

A surgical method employing an expanding device or bolt, such as the expanding bolt 100 of FIGS. 1-3, generally includes pre-forming a hole or tunnel (for example, a femoral tunnel) for insertion of the device. The expanding device 100 (attached to a graft) is then inserted into the hole/tunnel and then expanded. FIG. 4 illustrates an exemplary graft/plug assembly 200 comprising at least one graft tendon construct 199 tied to expanding plug 100 according to a method of the present invention. In the exemplary embodiment of FIG. 4, the graft construct 199 is formed as a continuous loop sutured together around expanding plug 100 (on a graft workstation, for example). The expanding plug 100 spaces the tendon strands apart to create a double-bundle configuration. Suture 75 is used to maintain placement of the tendon graft strands around the plug 100 and to create a desired length graft.

The graft/plug assembly 200 is installed into a tunnel (for example, femoral tunnel) by guiding the assembly oriented with graft-end 7 (FIG. 4) entering the tunnel and pulling the assembly up into the tunnel (in a first direction) using a passing strand (for example, suture 150). While the graft/plug assembly 200 is advanced in the first direction (i.e., pulled up into the tunnel or pushed up antegrade), the plug 100 is in the unexpanded configuration, with resilient arms 60a, 60b in the collapsed state, to allow the graft/plug assembly 200 to advance through the bone tunnel.

To deploy expansion plug 100, a second step in conducted, in which graft end 7 is pulled in a second direction (i.e., the joint line end 9 is pulled in a second direction, using another set of passing strands (for example, suture strands)). The load is applied in an opposite direction (for example, in a retrograde manner) to that described above for the initial first step when the plug is in a collapsed or undeployed configuration. Pulling on joint-line end 9 of the graft/plug assembly 200 in the second direction allows the deployment of expanding plug 100. Joint-line end 9 of expansion plug 100 is urged by the load being applied by pulling on the passing suture to allow the resilient arms 60a, 60b to deploy from the unexpanded configuration to the expanded or deployed configuration shown in FIGS. 1-3. Resilient arms 60a, 60b expand diametrically and radially to achieve interference fixation of soft tissue (for example, graft tendon) in the bone tunnel. In this manner, the arms will apply a lateral and a radial force, and will resiliently engage the surrounding bone to prevent the plug from being withdrawn from the bone tunnel in the direction of its entry.

Any expanding plastic polymer can be used for the expanding plug 100. Resilient arms 60a, 60b may be also formed out of a pseudoelastic shape memory alloy such as a nickel titanium alloy (for example, Nitinol). The use of such materials, in combination with the normal orientation of the arms relative to the anchor body, permits the arms to initially deflect inwardly to the extent required to permit the anchor to move forward in the bone tunnel, yet still resiliently "spring back" toward their normal, outwardly projecting position so as to prevent the anchor from withdrawing back out the bone tunnel.

The width of the plug and looped graft together preferably does not exceed about 10 mm, on average, with a double-wrapped autograft semitendinosis graft or a single tibialis tendon allograft. The width of the plug should be approximately 5 mm expanding to a minimum of 10 mm at body temperature. Maximum resistance of graft pullout in porcine bone sockets equal to the diameter of the graft preferably is not less than 500 N.

The present invention provides a low profile construction with the graft looped around the bottom and slots (through holes) on the side to accommodate the graft strands. It is not necessary to enlarge the overall bone tunnel size needed. Additional strands (for example, one to two no. 5 FiberWire sutures) may be attached to the base of the expandable plug for graft passing and backup fixation.

Advantageously, the expanding bolt 100 of the present invention is a self-reinforcing structure. The greater the load applied on the graft-end toward the knee, the more expanding and compression into the bone tunnel walls is achieved, resulting in higher pullout strength.

Although the present invention has been described in relation to particular embodiments thereof, many other variations and modifications and other uses will become apparent to those skilled in the art. Therefore, the present invention is to be limited not by the specific disclosure herein, but only by the appended claims.

## Claims

1. A device for soft tissue fixation comprising:
a body having a longitudinal axis, a distal end and a proximal end; and
two opposing resilient arms located at the proximal end, the arms being configured to expand resiliently radially to achieve interference fixation of a soft tissue graft inside of a bone tunnel.

2. The device of claim 1, wherein the body comprises a first main portion having a rectangular configuration and a second inner portion having a rectangular configuration, the width and the length of the first portion being greater than the width and the length of the second portion.

3. The device of claim 1, wherein the first main portion has a first plurality of through holes at its most distal end, and wherein the second portion has a second plurality of through holes.

4. The device of claim 1, wherein the arms have a first unexpanded condition and a second expanded condition, wherein the expanded width of the device is about 5% to about 50% greater that the unexpanded width of the device.

5. The device of claim 4, wherein the expanded width of the device is about 25% greater that the unexpanded width of the device.

6. The device of claim 1, wherein the body has an A-frame configuration.

7. The device of claim 1, wherein each of the arms is provided with an axial split to allow the arms to expand diametrically.

8. The device of claim 1, wherein the arms expand diametrically from a first diameter of about 5mm to a second diameter of about 10mm.

9. The device of claim 1, wherein the arms are formed of a polymer.

10. The device of claim 1, wherein the soft tissue graft is a tendon or a ligament.

11. The device of claim 1, wherein the arms are symmetrical relative to a longitudinal axis of the body and to the second end.

12. The device of claim 11, wherein the arms comprise a pseudoelastic shape memory alloy.

13. The device f claim 14, wherein the memory alloy is a nickel titanium alloy.
